# EUROPÄISCHE PATENTSCHRIFT

(11) **EP 1 591 080 B1**
(45) Veröffentlichungstag und Bekanntmachung des Hinweises auf die Patenterteilung: **08.08.2007**
(21) Anmeldenummer: 05008340.1
(22) Anmeldetag: 16.04.2005
(51) Int. Cl.: A61F 2/16

(54) **Intraokularlinse**
Intraocular Lens
Lentille intraoculaire

(30) Priorität: 30.04.2004 DE 102004021755
(43) Veröffentlichungstag der Anmeldung: 02.11.2005
(73) Patentinhaber: HumanOptics AG, 91054 Erlangen (DE)
(72) Erfinder: Messner, Arthur, Dr., 91220 Schnaittach (DE); Özmen, Ersan, 91054 Erlangen (DE); Use, Tim, 90469 Nürnberg (DE)
(74) Vertreter: Rau, Albrecht

(56) Entgegenhaltungen:
- EP-A- 0 579 528
- EP-A- 0 592 813
- US-A1- 2002 095 212
- US-A1- 2004 024 454
- US-B1- 6 409 762

## Beschreibung

Die Erfindung betrifft eine Intraokularlinse zur Implantation in den Kapselsack eines Auges, insbesondere eines menschlichen Auges.

Aus der WO 02/17818 A1 ist eine Intraokularlinse bekannt. Von dieser stehen jeweils zwei flexible Beine radial hervor, die über einen Steg miteinander verbunden sind. Die Beine und der Steg weisen im Wesentlichen dieselbe Dicke auf. Darüber hinaus sind keine definierten Gelenke vorgesehen. Das Kompressionsverhalten der Haptiken bei der Implantation in den Kapselsack ist deshalb nicht definiert.

Aus der EP 0 592 813 A1 ist eine weitere Intraokularlinse bekannt. Diese ist über mindestens einen von der Optik radial vorstehenden Verbindungssteg mit einer die Optik umgebenden, im Wesentlichen kreisförmig ausgebildeten Haptik verbunden. Dabei weist die Haptik zwei symmetrisch angeordnete Vorkehrungen auf, welche das Fassen der Linse erleichtern sollen. Bei dieser Linse ist eine dauerhafte Deformation der Haptik in Radialrichtung in der optischen Ebene nicht möglich.

Aus der US 6,409,762 B1 ist eine weitere Intraokularlinse bekannt. Diese weist jeweils zwei von der Optik radial vorstehende Stege auf, die über einen konvexen im Wesentlichen kreisbogenförmigen Verbindungssteg miteinander verbunden sind. Ein Zusammendrücken der Haptiken in radialer Richtung führt dazu, dass der Verbindungssteg sehr schnell an den radial vorstehenden Stegen anliegt, wie dies in Figur 3 der US 6,409,762 B1 gezeigt ist. Das radiale Deformationsintervall ist somit sehr begrenzt.

Der vorliegenden Erfindung liegt die Aufgabe zugrunde, eine Intraokularlinse zu schaffen, die nach der Implantation zentrisch im Kapselsack sitzt und die sich leicht an einen weiten Bereich verschiedener Kapselsackdurchmesser anpasst.

Die Aufgabe wird durch die Merkmale des Anspruchs 1 gelöst. Der Kern der Erfindung besteht darin, an einer Optik Haptiken vorzusehen, die aus Z-förmigen Haptikbügeln und einem diese verbindenden Verbindungsbügel bestehen. Der Verbindungsbügel ist konkav, das heißt von der optischen Achse aus gesehen nach innen gekrümmt. Je kleiner der Durchmesser des Kapselsacks ist, umso mehr werden die Z-förmigen Haptikbügel in Richtung auf den Rand der Optik gedrückt, wobei gleichzeitig der Verbindungsbügel stärker durchgebogen wird.

Weitere vorteilhafte Ausgestaltungen der Erfindung ergeben sich aus den Unteransprüchen.

Zusätzliche Merkmale und Einzelheiten der Erfindung ergeben sich aus der Beschreibung eines Ausführungsbeispiels anhand der Zeichnung. Es zeigen:
- Fig. 1: eine posteriore Ansicht der erfindungsgemäßen Intraokularlinse,
- Fig. 2: eine anteriore Ansicht der Intraokularlinse gemäß Fig. 1,
- Fig. 3: einen Querschnitt gemäß der Schnittlinie III-III in Fig. 1,
- Fig. 3a: eine Ausschnittvergrößerung eines Bereichs von Fig. 3, und
- Fig. 4: die Ansicht der Intraokularlinse gemäß Fig. 1 mit angedrückten Haptiken.

Eine einteilig ausgebildete Intraokularlinse 1 weist eine mittig angeordnete Optik 2 mit einer durch die Mitte der Optik und senkrecht zu dieser verlaufenden optischen Achse 3 auf. Die Optik weist ferner eine senkrecht zur Achse 3 mittig zur Optik 2 verlaufende Linsenebene 4 auf. Die Optik besteht aus einem transparenten, flexiblen Material, beispielsweise pHEMA, und besitzt die Eigenschaften einer optischen Linse. Die Optik 2 weist einen Durchmesser Mₒ auf, für den gilt: 5 mm ≤ Mₒ ≤ 8 mm, insbesondere Mₒ ≅ 6 mm. Die Optik 2 weist eine anteriore Oberfläche 5 sowie eine posteriore Oberfläche 6 auf. Im implantierten Zustand ist die anteriore Oberfläche 5 der Augenvorderkammer zugewandt, wohingegen die posteriore Oberfläche 6 der Netzhaut zugewandt ist. Die Optik 2 weist einen kreisförmigen Rand 7 auf.

Von dem Rand 7 der Optik 2 erstrecken sich zwei Haptiken 8, 9 in entgegengesetzte Richtungen, sind also am Rand 7 um 180° gegeneinander versetzt. Die Haptiken 8 und 9 sind bezüglich der Achse 3 punktsymmetrisch zueinander, das heißt die Haptik 8 ist durch eine Drehung um 180° um die Achse 3 in die Haptik 9 überführbar. Die Haptiken 8 und 9 dienen der Abstützung der Optik 2 im Kapselsack. Die Haptiken 8 und 9 weisen jeweils einen Haptikbügel 10 und einen Haptikbügel 11 auf, die vereinfacht ausgedrückt beide Z-förmig sind und sich vom Rand 7 nach außen und aufeinander zu erstrecken. Z-förmig im Sinne dieses Patents bedeutet, dass sowohl eine Struktur vorliegen kann, die im Wesentlichen der Form des Buchstabens Z entspricht, als auch eine Struktur vorliegen kann, die der Form eines gespiegelten Z entspricht, bei dem also der Mittelstrich nicht von rechts oben nach links unten verläuft, sondern von links oben nach rechts unten. Der Haptikbügel 10 besteht aus einem Innenbügel 12, einem sich daran anschließenden Mittelbügel 13 sowie einem sich daran anschließenden Außenbügel 14. Der Innenbügel 12 weist im Wesentlichen die Form des Teils eines Rings, der sich von einem Teil des Rands 7 mit einem Zentrumswinkel b nach außen erstreckt. Für den Winkel b gilt: 90° ≥ b ≥ 30°, insbesondere b ≅ 80°. Der Innenbügel 12 wird an beiden Enden durch Außen-Kanten 15, 16 begrenzt, die im Wesentlichen tangential zum Rand 7 verlaufen. Der Innenbügel 12 weist einen mittleren Bereich auf, der mit dem Mittelbügel 13 verbunden ist und eine angulare Breite c aufweist, für die gilt: 15° ≤ c ≤ 25°, insbesondere c ≅ 18°. In diesem Bereich weist der Innenbügel 12 eine radiale Breite B₁ auf, für die gilt: B₁ ≅ 0,16 x Mₒ / 2. Der Innenbügel 12 bildet sozusagen den unteren Schenkel des Buchstabens Z. Der Innenbügel 12 ist entlang des Winkels b einteilig mit dem entsprechenden Abschnitt des Randes 7 verbunden.

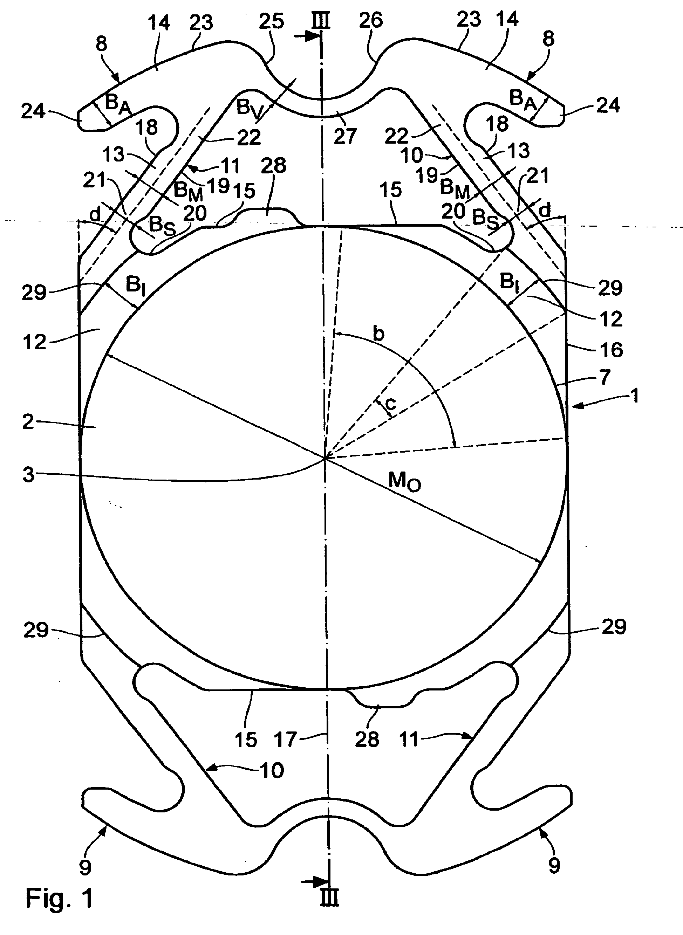

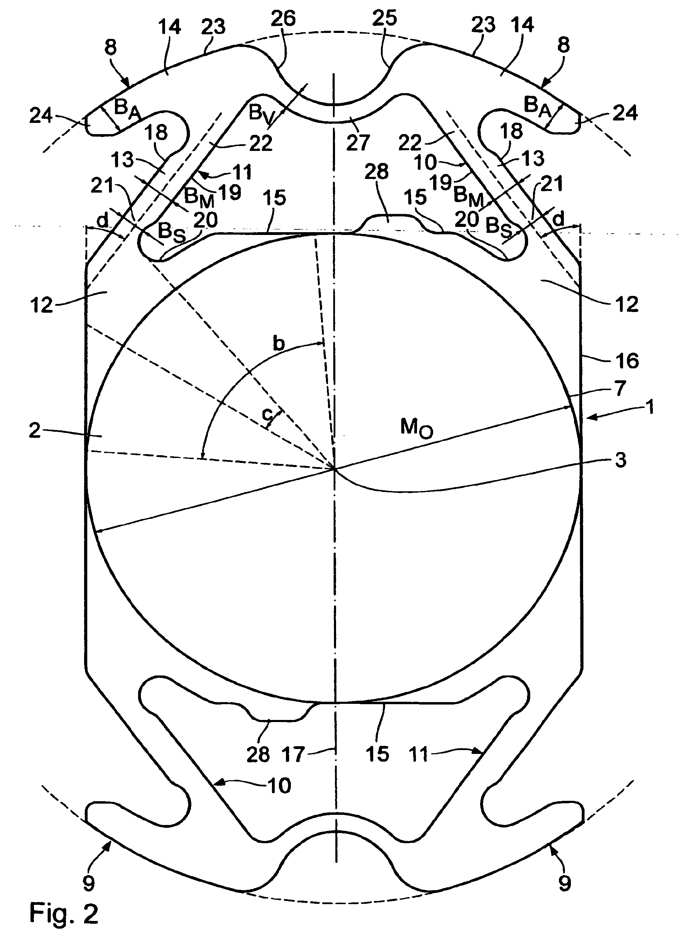

Von dem mittleren Bereich des Innenbügels 12 mit der angularen Breite c erstreckt sich der im Wesentlichen gradlinige Mittelbügel 13 schräg in Richtung auf die in Fig. 1 dargestellte, senkrecht zur Linsenebene 4 verlaufende Schnittebene 17, in der die optische Achse 3 liegt. Der Mittelbügel 13 schließt mit der Kante 16, die parallel zur Schnittlinie 17 verläuft, einen Winkel d ein, für den gilt: 25° ≤ d ≤ 60°, insbesondere d ≅ 36°. Der Mittelbügel 13 weist im Wesentlichen parallel zueinander verlaufende Seitenwände 18, 19 auf. Durch die Seitenwand 19 und die Kante 15 wird eine halbkreisförmige Ausnehmung 20 begrenzt, wodurch der Mittelbügel 13 im Bereich seines unteren Endes eine Sollbiegestelle 21 erhält, bei der das Material eine im Vergleich zur Umgebung geringere Breite Bₛ besitzt. Ansonsten besitzt der Mittelbügel 13 eine Breite B_{M}, für die gilt: B_{M} > Bₛ. Die Sollbiegestelle 21 übernimmt die Funktion eines Gelenks und wird auch als Innengelenk bezeichnet. Der Mittelbügel 13 entspricht dem schräg verlaufenden Abschnitt des Buchstabens Z. Im Bereich des äußeren Endes des Mittelbügels 13 befindet sich eine Sollbiegestelle 22, die auch als Außengelenk bezeichnet wird. Die Breite Bₛ im Bereich der Sollbiegestellen 21 und 22 beträgt ungefähr 0,27 mm. Die Breite des Materials im Bereich der Sollbiegestelle 22 ist ebenfalls geringer als die sonstige Breite B_{M}. Vom äußeren Ende des Mittelbügels 13 erstreckt sich der Außenbügel 14 in die der Schnittebene 17 abgewandte Richtung. Der Außenbügel 14 entspricht der oberen Querlinie des Buchstabens Z. Der Außenbügel 14 besitzt eine in radialer Richtung gesehen außenliegende Außenkontur 23, die im Wesentlichen auf einem Bogen um die Achse 3, insbesondere einem Kreisbogen, liegt, wie dies in Fig. 2 angedeutet ist. Der Außenbügel 14 weist ein vorstehendes freies Ende 24 auf, dessen Breite B_{A} ungefähr B_{M} entspricht. Durch das freie Ende 24 und den Mittelbügel 13 wird ein U-förmiger Freiraum begrenzt.

Der zur selben Haptik 8 gehörende Haptikbügel 11 ist, was den Mittelbügel 13 und den Außenbügel 14 betrifft, bezüglich der Schnittebene 17 spiegelsymmetrisch ausgebildet, so dass bezüglich der Details auf die Beschreibung des Haptikbügels 10 verwiesen wird. Der Haptikbügel 11 besitzt in der in Fig. 1 dargestellten Ansicht die Form eines Z, wohingegen der Haptikbügel 10 die Form eines an der Ebene 17 gespiegelten Z besitzt. Zwischen den der Ebene 17 zugewandten Enden 25 und 26 der beiden Außenbügel 14 ist ein Verbindungsbügel 27 angeordnet. Dieser ist bezüglich der Achse 3 konkav, das heißt nach innen gekrümmt, ausgebildet. Der Bügel weist im Wesentlichen eine konstante Krümmung auf. Der Verbindungsbügel 27 weist eine Breite Bᵥ auf, für die gilt: Bᵥ ≅ 0,2 mm. Die Breite Bᵥ des Verbindungsbügels 27 ist somit geringer als die Breite Bₛ der Sollbiegestellen 21, 22.

Die bezüglich der Ebene 17 gegenüberliegenden Innenbügel 12 unterscheiden sich insofern, als beim Haptikbügel 11 eine von der Kante 15 nach oben vorstehende, stegförmige Nase 28 vorgesehen ist, die als Orientierungsmittel dient. Bei der Haptik 9 ist die Nase 28 bezüglich der Achse 3 punktsymmetrisch angeordnet. Die Nase 28 dient als Orientierungsmittel, um bei der Implantation sicherzustellen, dass die Oberflächen 5 und 6 nicht vertauscht werden. Von hinten gesehen, das heißt wie in Fig. 1 dargestellt, muss die Nase 28 somit links oben sein. Betrachtet man die Linse 1 entsprechend Fig. 2 von vorne, so befindet sich die Nase 28 oben rechts. Als Orientierungsmittel kann jedes von außen erkennbare Merkmal verwendet werden, das bezüglich der Ebene 17 nicht spiegelsymmetrisch angeordnet ist. Die punktsymmetrische Anordnung des Orientierungsmittels erleichtert die Handhabung jedoch, da die beiden Haptiken 8 und 9 gleichwertig sind.

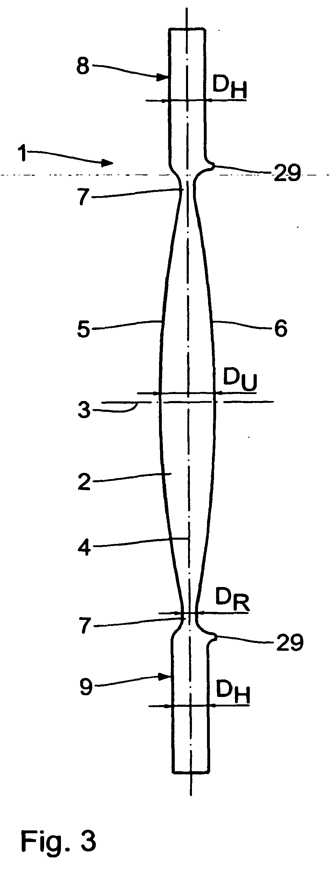

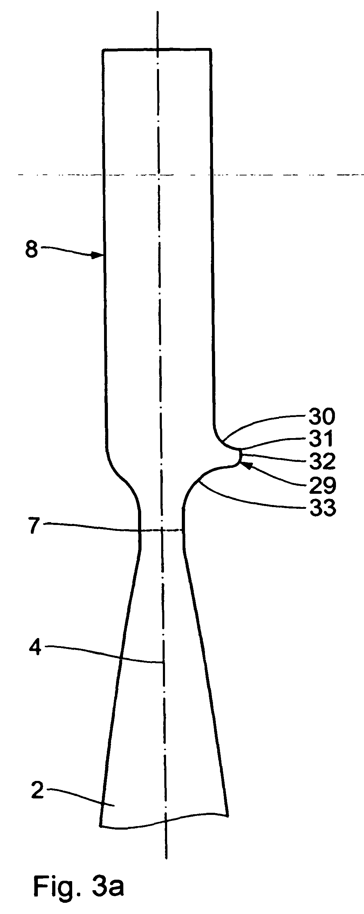

Wie aus Fig. 3 erkennbar ist, verlaufen die anteriore Oberfläche 5 und die posteriore Oberfläche 6 bezüglich der Linsenebene 4 konvex, das heißt sie sind nach außen gewölbt. Die Dicke D_{U} der Optik 2 im Bereich der Achse 3 hängt von der erforderlichen Brechkraft der Linse ab. Die Dicke nimmt von der Mitte hin zum Rand 7 kontinuierlich ab. Im Bereich des Randes ergibt sich ein Bereich D_{R} minimaler Dicke, für den gilt: D_{R} ≅ 0,18 mm. Die sich an den Rand 7 anschließenden Haptiken 8 und 9 weisen in axialer Richtung eine konstante Dicke D_{H} auf, für die gilt: D_{H} ≅ 0,44 mm. Es gilt somit: 1,5 ≤ D_{H} / D_{R} ≤ 3,5, insbesondere D_{H} / D_{R} ≅ 2,5. Bemerkenswert ist, dass die Dicke D_{H} der Haptiken 8, 9 im Bereich der Sollbiegestellen 21, 22 größer ist als die Breite Bₛ der Haptikbügel 10, 11 im Bereich der Sollbiegestellen 21, 22. Es gilt: 1,2 ≤ D_{H} / B_{S} ≤ 2,5, insbesondere D_{H} / B_{S} ≅ 1,7. Die Dicke D_{H} des Verbindungsbügels 27 ist größer als dessen Breite Bᵥ. Es gilt: 1,5 ≤ D_{H} / B_{V} ≤ 3, insbesondere D_{H} / B_{V} ≅ 2,2. Die Tatsache, dass im Bereich der flexiblen Elemente, das heißt der Sollbiegestellen 21 und 22 sowie des Verbindungsbügels 27, die Dicke des Materials größer ist als dessen Breite, stellt sicher, dass bei einer Deformation in Reaktion auf Kräfte, die radial von außen auf die Haptiken 8 und 9 wirken, die Haptiken 8, 9 in der Linsenebene 4 verformt werden und die Optik 2 nicht in die posteriore Richtung oder die anteriore Richtung ausbricht.

Auf der posterioren Seite der Linse 1, das heißt in Fig. 3 rechts, befindet sich zwischen dem Rand 7 und den Haptiken 8 bzw. 9 eine axial vorstehende Zellbarriere 29. Die Zellbarriere 29 hat die Form eines Abschnitts eines Kreisbogens und liegt im gesamten Übergangsbereich von dem Innenbügel 12 zum Mittelbügel 13. Die Zellbarriere 29 weist radial von außen nach innen gesehen eine kontinuierlich ansteigende Flanke 30 konstanter Krümmung auf. An dem höchsten Punkt der Barriere 29 endet die Flanke 30 unter Bildung einer scharfen Kante 31. Von der Kante 31 ausgehend weist die Barriere 29 von der Ebene 4 aus gesehen einen konvexen Abschnitt 32 mit konstanter Krümmung auf, der kontinuierlich in einen konkaven Abschnitt 33 mit einer anderen, aber im Wesentlichen wieder konstanten Krümmung übergeht. Die Zellbarriere 29 verhindert, dass sich Linsenepithelzellen, die nach der Entfernung der natürlichen Linse aus dem Kapselsack in letzterem verblieben sind, nach der Implantation der Linse 1 vom Äquatorialbereich des Kapselsacks nach innen parallel zur Oberfläche der Haptiken 8 und 9 bis zur Optik 2 ausbreiten und zu einer Abnahme der Transparenz im Bereich der Optik führen. Spätestens an der Barriere 29 werden sie gestoppt. Von besonderer Bedeutung ist hierbei die scharfe Kante 31. Zur Vermeidung unerwünschten Zellwachstums ist es möglich, alle Außenkanten der Intraokularlinse scharfkantig auszubilden.

Im Folgenden wird die Implantation der Intraokularlinse 1 mit Blick auf die erforderlichen Deformationseigenschaften der Linse beschrieben. Die Figuren 1 bis 3a zeigen die Intraokularlinse 1 in spannungsfreiem Zustand. Die Intraokularlinse 1 wird beispielsweise aus einem transparenten Acrylat durch spanende Bearbeitung hergestellt. Bei der Bearbeitung ist das Material hart.

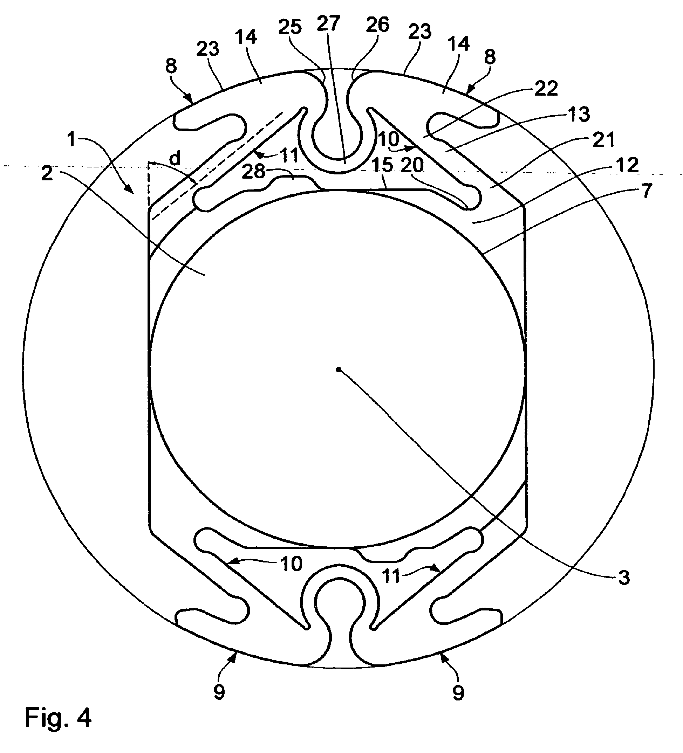

Anschließend wird das Zwischenprodukt in eine wässrige Kochsalzlösung gegeben, wodurch das Acrylat Wasser aufnimmt und elastisch wird. Dieses Material wird als pHEMA, Polyhydroxyethylmethacrylat, bezeichnet. Es ist reversibel verformbar. Es können auch andere Materialien verwendet werden. Dies bedeutet, dass das Material nach einer Deformation, z. B. durch ein Zusammendrücken der Haptiken, nach dem Wegfall der äußeren Einwirkung wieder den ursprünglichen Zustand einnimmt. In den Fig. 1 bis 3a ist der spannungsfreie Zustand einer bereits elastischen Intraokularlinse 1 dargestellt. Kommt es krankheitsbedingt zu einer Trübung der natürlichen Linse im menschlichen Auge, so wird diese unter Erhaltung des die natürliche Linse umgebenden Kapselsacks entfernt. Hierfiir wird im Kapselsack eine kreisförmige Öffnung angebracht. Zur Implantation wird die in den Fig. 1 bis 3a dargestellte Linse 1 zusammengedrückt, durch die Öffnung in den Kapselsack eingeführt und dehnt sich dann aus, bis die Außenkonturen 23 der Haptiken 8 und 9 am inneren Rand 34 des Kapselsacks in dessen Äquatorialbereich zu liegen kommen, wie dies in Fig. 4 dargestellt ist.

Ein Vergleich der Fig. 1 und 4 zeigt das Deformationsverhalten der Haptiken 8 und 9, wenn auf diese von außen in radialer Richtung Kräfte wirken. Die Sollbiegestelle 21 sorgt dafür, dass der Mittelbügel 13 auf die Ebene 17 hin verschwenkt wird, ohne selber seine im Wesentlichen gerade Form zu verlieren. Hierdurch wird der Winkel d vergrößert. Die Sollbiegestelle 22 stellt sicher, dass eine Verschwenkung des Mittelbügels 13 relativ zum Außenbügel 14 im Bereich der Sollbiegestelle 22 möglich ist, ohne dass die beiden Bügel 13, 14 deformiert werden. Die Außenkontur 23 der Außenbügel 14 liegt somit auch in dem in Fig. 4 dargestellten deformierten Zustand flächig an dem kreisförmigen Rand 34 des Kapselsacks an. Das Zusammendrücken der Haptiken 8 und 9 führt ferner dazu, dass der Verbindungsbügel 27 stärker durchgebogen wird und zunehmend fast die Form eines Halbkreises erhält. Die vergleichsweise geringe Breite Bᵥ des Verbindungsbügels 27 stellt sicher, dass dieser deformiert wird und nicht die anderen Teile in seinem Umfeld. Insgesamt hat die Ausgestaltung der Haptiken 8 und 9 den Vorteil, dass die Intraokularlinse 1 für Kapselsäcke mit verschiedenen Innendurchmessern verwendet werden kann, ohne dass die Gefahr der axialen Verkippung der Optik besteht. Das vordere und hintere Kapselblatt kann durch die Öffnung in jeder Haptik miteinander verkleben, wodurch eine zusätzliche Fixierung der Intraokularlinse 1 gewährleistet wird.

## Patentansprüche

1. Intraokularlinse zur Implantation in den Kapselsack eines Auges
a. mit einer Optik (2), welche aufweist
i. einen im Wesentlichen kreisförmigen Rand (7),
ii. eine optische Achse (3), und
iii. eine senkrecht zur optischen Achse (3) verlaufende Linsenebene (4),
b. mit mindestens zwei an der Optik (2) befestigten, einteilig mit der Optik (2) ausgebildeten, aus einem reversibel verformbaren Material bestehenden Haptiken (8, 9) zur Abstützung der Optik (2) gegenüber dem Kapselsack, wobei jede Haptik (8, 9) aufweist
i. einen am Rand (7) angelenkten, Z-förmigen ersten Haptikbügel (10)und
ii. einen am Rand (7) angelenkten, Z-förmigen zweiten Haptikbügel (11),
**dadurch gekennzeichnet, dass**
c. die Haptikbügel (10,11) jeweils einen Innenbügel (12) aufweisen, der im Wesentlichen die Form des Teils eines Rings aufweist, der sich von einem Teil des Randes (7) mit einem Zentrumswinkel b nach außen erstreckt, wobei gilt: 90° ≥ b ≥ 30°,
d. die Innenbügel (12) der Haptikbügels (10,11) an den Rand (7) der Optik (2) angrenzen und entlang des Winkels b einteilig mit dem entsprechenden Abschnitt des Randes (7) verbunden sind,
e. jede Haptik (8,9) einen den ersten Haptikbügel (10) und den zweiten Haptikbügel (11) verbindenden, bezogen auf die optische Achse (3) konkaven Verbindungsbügel (27) aufweist, und
f. die Innenbügel (12) durch Außenkanten (15, 16) begrenzt werden, die im Wesentlichen tangential zum Rand (7) verlaufen.

2. Intraokularlinse gemäß Anspruch 1, **dadurch gekennzeichnet, dass** die Intraokularlinse (1) bezüglich der optischen Achse (3) punktsymmetrisch ist.

3. Intraokularlinse gemäß Anspruch 1 oder 2, **dadurch gekennzeichnet, dass** die Haptikbügel (10, 11) aus einem Innenbügel (12), einem Mittelbügel (13) und einem Außenbügel (14) bestehen.

4. Intraokularlinse gemäß Anspruch 3, **dadurch gekennzeichnet, dass** der Mittelbügel (13) an den Innenbügel (12) über ein Innengelenk (21) angelenkt ist.

5. Intraokularlinse gemäß Anspruch 4, **dadurch gekennzeichnet, dass** der Außenbügel (14) an dem Mittelbügel (13) über ein Außengelenk (22) angelenkt ist.

6. Intraokularlinse gemäß Anspruch 5, **dadurch gekennzeichnet, dass** der Außenbügel (14) eine radial außenliegende Außenkontur (23) besitzt, die im Wesentlichen auf einem Bogen um die optische Achse (3) liegt.

7. Intraokularlinse gemäß einem der vorangehenden Ansprüche, **dadurch gekennzeichnet, dass** der Verbindungsbügel (27) eine axiale Dicke Dᵥ und in der Linsenebene (4) eine Breite Bᵥ besitzt, für die gilt:
Dᵥ > Bᵥ.

8. Intraokularlinse gemäß einem der vorangehenden Ansprüche, **dadurch gekennzeichnet, dass** die Haptiken (8, 9) quer, insbesondere senkrecht, zur Linsenebene (4) nach außen vorstehende stegförmige Zellbarrieren (29) zur Vermeidung von Zellmigrationen aufweisen.

9. Intraokularlinse gemäß einem der vorangehenden Ansprüche, **dadurch gekennzeichnet, dass** asymmetrisch angeordnete Orientierungs-Mittel (28) zur eindeutigen Platzierung der Intraokularlinse (1) im Kapselsack vorgesehen sind.

10. Intraokularlinse gemäß einem der vorangehenden Ansprüche, **dadurch gekennzeichnet, dass** der erste Haptikbügel (10) über ein erstes Innengelenk (21) am Rand (7) angelenkt ist und/oder der zweite Haptikbügel (11) über ein zweites Innengelenk (21) am Rand (7) angelenkt ist.

## Claims

1. An intraocular lens for implantation in the capsular bag of an eye, comprising
a. an optic (2) which has
i. a substantially circular rim (7),
ii. an optical axis (3), and
iii. a lens plane (4) oriented perpendicularly to the optical axis (3);
b. at least two haptics (8, 9) for support of the optic (2) against the capsular bag, the haptics (8, 9) being fixed to the optic (2) and forming one piece with the optic (2) and consisting of reversibly deformable material, each haptic (8, 9) having
i. a first Z-shaped haptic clamp (10) articulated to the rim (7),
ii. a second Z-shaped haptic clamp (11) articulated to the rim (7),
**characterised in that**
c. the haptic clamps (10, 11) comprise an inner clamp (12) respectively, which has substantially the shape of the part of a ring that extends from part of the rim (7) outwards by an angle at centre b, to which applies: 90° ≥ b ≥ 30°,
d. the inner clamps (12) of the haptic clamps (10, 11) adjoin the rim (7) of the optic (2) and are integrally united with the corresponding section of the rim (7) along the the angle b,
e. each haptic (8, 9) comprises a connecting clamp (27) being concave in relation to the optical axis (3) and linking the first haptic clamp (10) and the second haptic clamp (11), and
f. the inner clamps (12) are defined by outer edges (15, 16) being substantially tangential to the rim (7).

2. An intraocular lens according to claim 1, **characterised in that** the intraocular lens (1) is point-symmetric in relation to the optical axis (3).

3. An intraocular lens according to claim 1 or 2, **characterised in that** the haptic clamps (10, 11) are comprised of an inner clamp (12), a central clamp (13) and an outer clamp (14).

4. An intraocular lens according to claim 3, **characterised in that** the central clamp (13) is articulated to the inner clamp (12) by an inner joint (21).

5. An intraocular lens according to claim 4, **characterised in that** the outer clamp (14) is articulated to the central clamp (13) by an outer joint (22).

6. An intraocular lens according to claim 5, **characterised in that** the outer clamp (14) has a radially outward outer contour (23) which is located substantially on an arc about the optical axis (3).

7. An intraocular lens according to one of the preceding claims, **characterised in that** the connecting clamp (27) has an axial thickness Dᵥ and, in the lens plane (4), a width Bᵥ to which applies Dᵥ > Bᵥ.

8. An intraocular lens according to one of the preceding claims, **characterised in that** the haptics (8, 9) comprise cell barriers (29) in the form of ribs that project outwards crosswise, in particular perpendicularly, of the lens plane (4), serving to prevent cell migration.

9. An intraocular lens according to one of the preceding claims, **characterised in that** orienting means (28) of asymmetrical arrangement are provided, distinctly positioning the intraocular lens (1) in the capsular bag.

10. An intraocular lens according to one of the preceding claims, **characterised in that** the first haptic clamp (10) is articulated to the rim (7) by a first inner joint (21) and/or that the second haptic clamp (11) is articulated to the rim (7) by a second inner joint (21).

## Revendications

1. Lentille intra-oculaire pour une implantation dans le sac capsulaire d'un oeil
a. avec une optique (2) qui comprend
i. un bord sensiblement circulaire (7),
ii. un axe optique (3), et
iii. un plan de lentille (4) s'étendant perpendiculairement à l'axe optique (3),
b. avec au moins deux haptiques (8, 9) en un matériau à déformation réversible, réalisées en une seule partie avec l'optique (2), fixées à l'optique (2), pour l'appui de l'optique (2) par rapport au sac capsulaire, chaque haptique (8, 9) comportant :
i. une première boucle d'haptique en forme de Z (10) articulée sur le bord (7) et
ii. une seconde boucle d'haptique en forme de Z (11) articulée sur le bord (7),
**caractérisée en ce que**
c. les boucles d'haptiques (10, 11) comportent chacune une boucle intérieure (12) qui présente sensiblement la forme de la partie d'un anneau qui s'étend à partir d'une partie du bord (7) avec un angle central b vers l'extérieur, sachant que : 90° ≥ b ≥ 30°,
d. les boucles intérieures (12) des boucles d'haptiques (10, 11) sont contiguës au bord (7) de l'optique (2) et sont reliées en une seule partie au tronçon correspondant du bord (7) le long de l'angle b,
e. chaque haptique (8, 9) comporte une boucle de liaison (27) concave par rapport à l'axe optique (3) qui relie la première boucle d'haptique (10) et la seconde boucle d'haptique (11), et
f. les boucles intérieures (12) sont délimitées par des bords externes (15, 16) qui s'étendent sensiblement tangentiellement au bord (7).

2. Lentille intra-oculaire selon la revendication 1, **caractérisé en ce que** la lentille intra-oculaire (1) présente une symétrie ponctuelle par rapport à l'axe optique (3).

3. Lentille intra-oculaire selon la revendication 1 ou 2, **caractérisé en ce que** les boucles d'haptiques (10, 11) sont constituées d'une boucle intérieure (12), d'une boucle centrale (13) et d'une boucle extérieure (14).

4. Lentille intra-oculaire selon la revendication 3, **caractérisé en ce que** la boucle centrale (13) est articulée sur la boucle intérieure (12) au moyen d'une articulation intérieure (21).

5. Lentille intra-oculaire selon la revendication 4, **caractérisé en ce que** la boucle extérieure (14) est articulée sur la boucle centrale (13) au moyen d'une articulation extérieure (22).

6. Lentille intra-oculaire selon la revendication 5, **caractérisé en ce que** la boucle extérieure (14) présente un profil extérieur (23) placé radialement à l'extérieur qui se trouve sensiblement sur un arc autour de l'axe optique (3).

7. Lentille intra-oculaire selon l'une quelconque des revendications précédentes, **caractérisé en ce que** la boucle de liaison (27) présente une épaisseur axiale Dᵥ et une largeur Bᵥ dans le plan de lentille (4) auxquelles s'applique : Dᵥ > Bᵥ.

8. Lentille intra-oculaire selon l'une quelconque des revendications précédentes, **caractérisé en ce que** les haptiques (8, 9) comportent des barrières de cellules (29) en forme de nervures qui font saillie vers l'extérieur transversalement, en particulier perpendiculairement, au plan de lentille (4) pour empêcher les migrations de cellules.

9. Lentille intra-oculaire selon l'une quelconque des revendications précédentes, **caractérisé en ce qu'**il est prévu des moyens d'orientation (28) disposés asymétriquement pour le positionnement univoque de la lentille intra-oculaire (1) dans le sac capsulaire.

10. Lentille intra-oculaire selon l'une quelconque des revendications précédentes, **caractérisé en ce que** la première boucle d'haptique (10) est articulée sur le bord (7) au moyen d'une première articulation intérieure (21) et/ou la seconde boucle d'haptique (11) est articulée sur le bord (7) au moyen d'une seconde articulation intérieure (21).
